# EUROPEAN PATENT SPECIFICATION

(11) **EP 3 235 898 B1**
(45) Date of publication and mention of the grant of the patent: **03.03.2021**
(21) Application number: 17161399.5
(22) Date of filing: 16.03.2017
(51) Int. Cl.: C12M 1/107, C12M 1/26, C12M 1/34

(54) **DEVICE FOR PROBING THE CONTENTS OF TANKS OF ANAEROBIC DIGESTION SYSTEMS**
SAMPLINGVORRICHTUNG ZUM PRÜFEN DEN INHALT EINES ANAEROBEN GÄRTANKS
DISPOSITIF D'ÉCHANTILLONAGE POUR TESTER LE CONTENU DE LA CUVE D'UN SYSTÈME DE DIGÉSTION ANAÉROBIE.

(30) Priority: 21.04.2016 IT UA20162779
(43) Date of publication of application: 25.10.2017
(73) Proprietor: Bietifin S.R.L., 40138 Bologna (IT)
(72) Inventor: LANZI, Andrea, 26020 ACQUANEGRA CREMONESE (CREMONA) (IT); STOCKER, Guenther, 39049 VIPITENO (BOLZANO) (IT); PIRANI, Tommaso, 44122 FERRARA (IT)
(74) Representative: Casadei, Barbara

(56) References cited:
- CA-A- 965 260
- US-A- 2 137 128
- US-A- 4 563 896
- US-A- 5 094 113
- US-A- 5 537 881
- US-A- 5 878 813
- DATABASE WPI Week 201533 Thomson Scientific, London, GB; AN 2015-28738L XP002765627, -& JP 2015 085309 A (SHINKO PANTEC CO. LTD.) 7 May 2015 (2015-05-07)
- DATABASE WPI Week 200866 Thomson Scientific, London, GB; AN 2008-L20255 XP002765628, -& CN 200 992 553 Y (CHE A) 19 December 2007 (2007-12-19)

## Description

This invention relates to a device for probing the contents of tanks of anaerobic digestion systems.

Generally speaking, an anaerobic digestion system comprises one or more tanks in which are introduced natural substances which, in the absence of oxygen and at a controlled temperature, are degraded by a multiplicity of bacteria.

The degradation of natural substances produces biogas, which is a resource that can be used to power, for example, cogeneration, digestate and thermal systems.

The economic balance of an anaerobic digestion system is therefore strictly correlated to the products derived from the degradation of the natural substances contained in the tank and it is for this reason that the fermenting space of the tank is an important parameter of this balance.

During operation of an anaerobic digestion system it is found that the presence of any layers of fibrous material, either floating or deposited below the surface of the tank, and any layers of solid material or layers of muddy material, deposited on the bottom of the tank, reduce over time the fermenting volume of the tank with a consequent worsening of the economic balance of the system.

Currently, the tanks of know type can have one or more visual inspection windows which, however, do not allow the size of any layers of fibrous material or solid material or muddy material to be evaluated, in order to asses the need to restore the original fermenting volume of the tank US4563896 discloses a gauger's system for use in sampling bottom sediment conditions in an oil storage tank.

JP2015085309 discloses a method for estimating deposition state of sediment in methane fermentation tank by injecting a pressure type depth indicator towards a bottom surface from the upper part of a methane fermentation tank.

In this context, an aspect of the invention is to provide a device for probing the contents of the tanks of anaerobic digestion systems as disclosed in claim 1, comprising means for sampling the contents of the tank, which can be inserted into and removed from the tank, configured for detecting any layer of fibrous material, in particular floating or positioned under the surface of the tank, or any layer of solid or muddy material, in particular deposited on the bottom of the tank, and means for moving the sampling means configured for moving the sampling means along a vertical direction which is orthogonal to the bottom wall of the tank.

Advantageously, the sampling means allow the contents of the tank to be probed for assessing the actual work space of the tank and for assessing the biological state of the contents of the tank in a predetermined sampling area.

Further features and advantages of the invention are more apparent in the detailed description below, with reference to some preferred, non-limiting, embodiments of a device for probing the contents of tanks of anaerobic digestion systems as illustrated in the accompanying drawings, in which:
- Figure 1 is a schematic front view of the device according to the invention installed at a lateral wall of a tank of an anaerobic digestion system;
- Figure 2 is a schematic top view of the tank of Figure 1 indicating a sampling area of the device according to the invention;

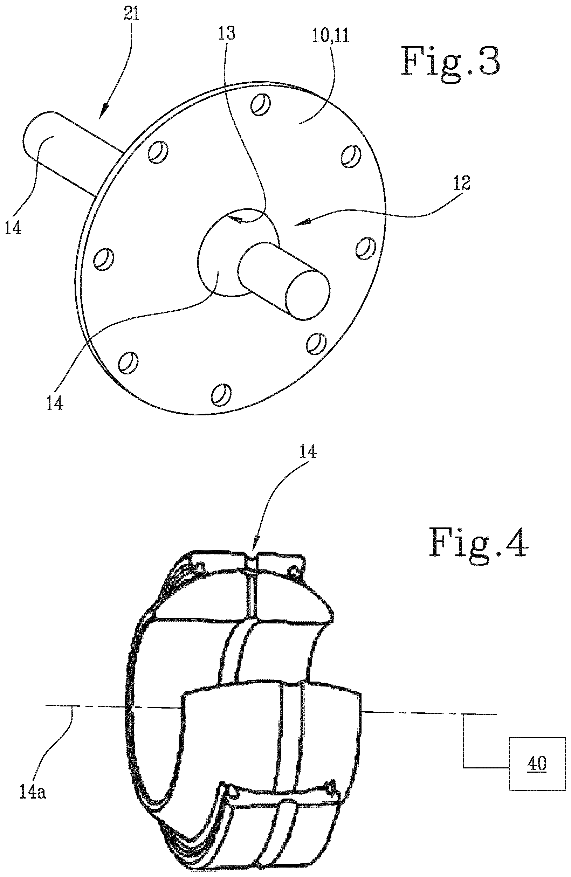

- Figure 3 is a schematic perspective view of the installation means and at least part of the positioning means of the device according to the configuration of Figure 1;
- Figure 4 is a schematic scaled-up perspective view of a detail of Figure 3;
- Figure 5 is a scaled-up detail of the installation means as illustrated in Figure 1;
- Figure 6 is a schematic front view of the device according to the invention installed at a top wall of a tank of an anaerobic digestion system;
- Figure 7 is a schematic front view of the device according to the invention during the operations for probing the bottom wall of the tank;
- Figure 8 is a schematic front view of a first variant of the sampling means of the device according to the invention in a non-operating configuration;
- Figure 9 is a schematic front view of a first variant of the sampling means of the device according to the invention in an operating configuration;
- Figure 10 is a schematic front view of a second variant of the sampling means of the device according to the invention in a non-operating configuration;
- Figure 11 is a schematic front view of a second variant of the sampling means of the device according to the invention in an operating configuration.

The numeral 1 denotes a device for probing the contents of tanks 2 of anaerobic digestion systems according to the invention.

The term tank 2 of an anaerobic digestion system means, for example, a pre-tank, of an open or closed type, a fermenting device, closed with a slab and/or closed with a textile cover, a post-fermenting device, closed with a slab or with a textile cover, or a storage tank, of an open or closed type.

The tank 2 is configured to house substances of natural origin, such as, for example, manure, sewage, chicken manure, dairy whey, vegetable waste, agricultural by-products, defining the fermenting liquid or contents 8 of the tank 2.

The substances of natural origin contained in the tank 2 can result in layers 44 of fibrous material, either floating or positioned under the surface of the tank 2, or layers 9 of solid material or layers 45 of a muddy type, deposited on the bottom wall 4 of the tank 2.

Generally speaking, the layers 45 of a muddy type cover at least partly the layers 9 of solid material.

Generally speaking, the tank 2 of an anaerobic digestion system is cylindrical in shape.

The tank 2 has at least one or more lateral walls 3 and a bottom wall 4. With reference in particular to the tanks 2 of the closed type, the tank 2 has a top wall 43.

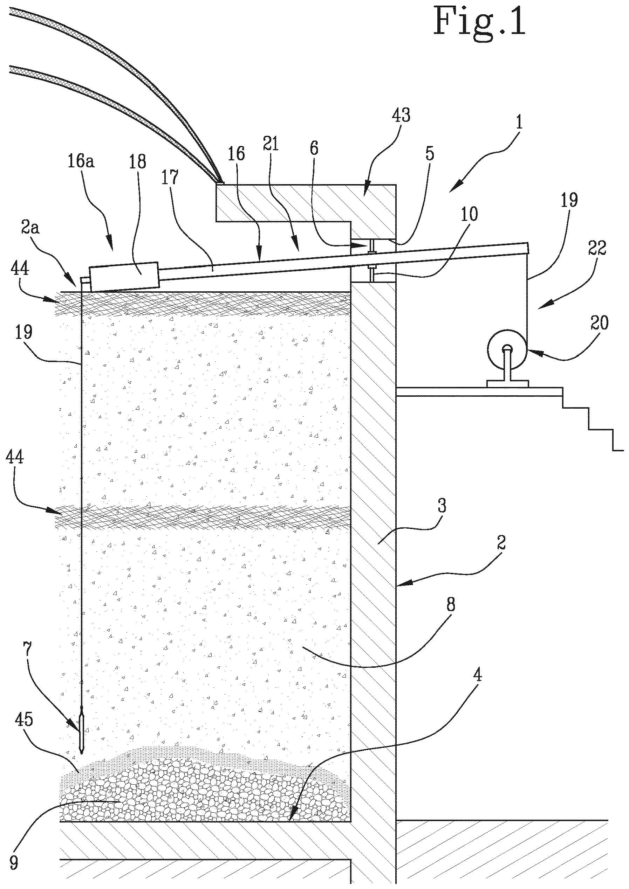

With reference to the embodiment illustrated in Figure 1, the tank 2 has at least one through opening 5 positioned at the lateral wall 3.

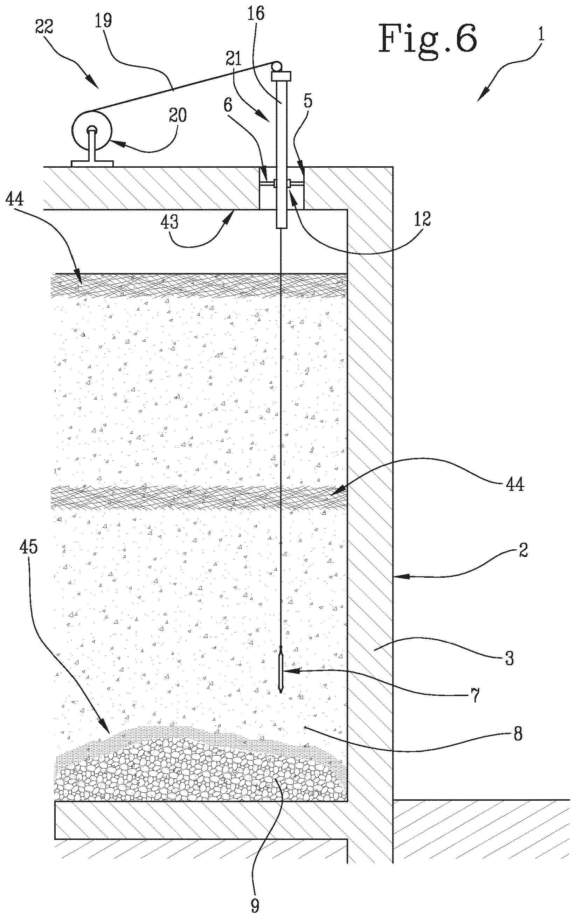

With reference to the embodiment illustrated in Figure 6, the tank 2 has a top wall 43 equipped with at least through an opening 5.

The device 1 comprises installation means 6 which connect the device 1 to the tank 2 to be inspected.

The installation means 6 are configured for being positioned in a respective through opening 5 of the tank 2.

It should be noted that the through opening 5, in which the installation means 6 are located, may be either positioned along the lateral wall 3 of the tank 2, as shown in Figure 1, or along the top wall 43 of the tank 2, as shown in Figure 6.

The installation means 6 comprise an anchoring element 10 configured for being inserted in the through opening 5 of the tank 2.

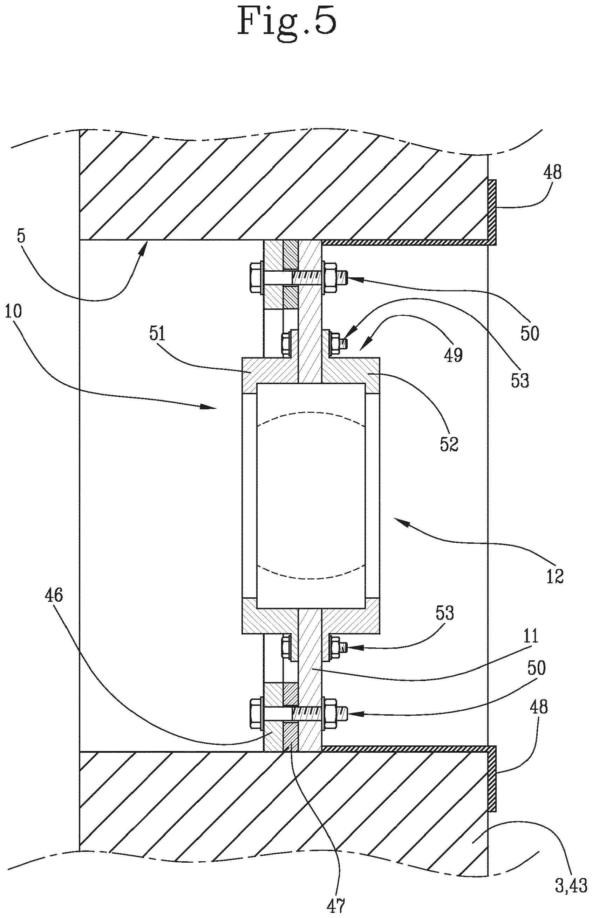

The anchoring element 10 comprises a first and a second flange 11 and 46 and a sealing element 47 interposed between them, as illustrated in Figure 5.

The first and second flange 11 and 46 are connected to each other by connecting means 50, in particular removable.

Preferably the connecting means 50 are in the form of bolts.

The fastening of the connecting means 50 compresses the gasket 47 interposed between the first and the second flange 11 and 46, ensuring the seal against the walls defining the through opening 5.

In order to avoid accidental falling of the installation means 6 in the contents 8 of the tank 2, the installation means 6 comprise one or more elements 48 for fixing the anchoring element 10 to the tank 2.

The fixing elements 48 are in the form of brackets.

The fixing elements 48 are connected to the first flange 11, in particular by means of the connecting means 50.

The device 1 comprises means 7 for sampling the contents 8 of the tank 2. The sampling means 7 are configured to detect any layer 44 of fibrous material, in particular floating or positioned under the surface of the tank 2, or any layer 9 of solid material or a layer 45 of a muddy type, in particular deposited on the bottom wall 4 of the tank 2, as schematically illustrated in Figure 1.

The device 1 comprises means 21 for positioning the sampling means 7 in a predetermined sampling area 2a inside the tank 2 relative to the perimeter defined by one or more side walls 3 of the tank 2.

The sampling means 7 can be introduced into and removed from the tank 2 using the positioning means 21.

The positioning means 21 are configured to be inserted and removed by means of the installation means 6, in particular by means of the anchoring element 10.

The positioning means 21 comprise at least one element 16 for guiding the sampling means 7.

The extension of the guide element 16 determines the positioning of the sampling means 7 inside the tank 2 relative to the through opening 5.

In particular, the sampling means 7 can be introduced into and removed from the guide element 16 in order to be inserted into and removed from the contents 8 of the tank 2.

In the embodiment described, the guide element 16 means a hollow pipe 17 having a passage section with dimensions such as to allow the insertion of the sampling means 7 inside it.

Alternatively, the guide element 16 comprises a plurality of pipes 17 connected one after the other, preferably using a male and female screw coupling, cables inside them having a respective passage section with dimensions such as to allow the insertion of the sampling means 7.

The plurality of pipes 17 allows the extension of the guide element 16 to be changed in relation to the predetermined sampling area 2a.

To prevent the guide element 16 immersing even partially in the contents 8 the tank 2, the positioning means 21 comprise at least one floating element 18 connected to a respective portion 16a of the guide element 16.

More specifically, the floating element 18 is connected to the guide element 16 in the vicinity of the relative end portion 16a designed to be inserted inside the tank 2.

The positioning means 21 comprise means 12 for orienting the sampling means 7 inside the tank 2.

Advantageously, the orientation means 12 allow the positioning of the sampling means 7 in predetermined sampling areas 2a of the tank 2 relative to a fixed reference element.

It should be noted that the sampling areas 2a of the tank 2 are determined from a predetermined sampling diagram depending on the dimensions of the tank 2 and any presence of one or more means for mixing the contents 8 of the tank 2, not illustrated.

The orientation means 12 comprise an element 14 oscillating about a main axis of rotation 14a.

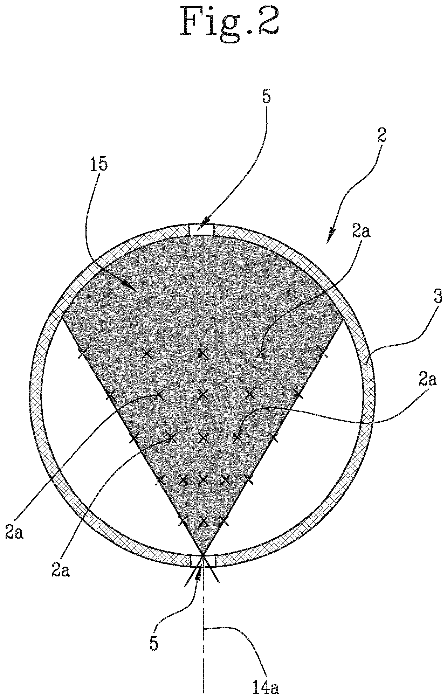

The oscillating element 14 is movable from a first to a second end position delimiting a sampling area 15 of the tank 2, as schematically illustrated in Figure 2. The sampling area 15 of the tank 2 comprises the above-mentioned sampling areas 2a.

Preferably, the main axis of rotation 14a defines the above-mentioned fixed reference element relative to which is defined the sampling area 2a, in particular its angular position, in the sampling area 15.

Preferably, the oscillating element 14 is a ball joint.

The orientation means 12 comprise a device 40 for detecting the angulation of the oscillating element 14, relative to a fixed reference element, in order to correctly position the sampling means 7 in the respective sampling area 2a.

The device 40 for measuring the angle of the oscillating element 14 is, for example, a digital protractor.

The installation means 6 comprise a seat 13 for housing at least part of the orientation means 12.

More specifically, the anchoring element 10 has the seat 13 for housing the orientation means 12.

More specifically, the first flange 11 has the seat 13 for housing the orientation means 12.

The seat 13 for housing the orientation means 12 is defined by a respective retaining body 49 of the oscillating element 14.

The retaining body 49 is connected to the first flange 11.

The retaining body 49 is inserted at least partly in the second flange 46.

The retaining body 49 comprises a first and a second element 51 and 52 connected to each other, in particular by connecting means 53, in particular removable.

The first and the second element 51 and 52 are positioned on opposite sides with respect to the first flange 11.

The first and the second element 51 and 52 are connected to the first flange 11 by the connecting means 53.

More specifically, the connecting means 53 are supported by the first flange 11.

Preferably, the connecting means 53 are in the form of bolts.

The positioning means 21 can be introduced and removed through the orientation means 12.

More specifically, the guide element 16 can be inserted and removed through the oscillating element 14.

In order to increase the angular excursion of the oscillating element 14, and consequently increase the extension of the sampling area 15, the orientation means 12 comprise a bushing, not illustrated, interposed between the guide element 16 and the oscillating element 14.

According to the invention, the device 1 comprises means 22 for moving the sampling means 7 configured to place inside the tank 2 the sampling means 7 along a vertical direction, orthogonal to the bottom wall 4 of the tank 2.

In this way, once the sampling means 7 are positioned inside the tank 2 in the predetermined sampling area 2a, it is possible to position the sampling means 7 according to one or more depths which are different to each other relative to the bottom wall 4 of the tank 2.

The movement means 22 are configured to measure the depth of the sampling means 7 inside the tank 2.

The term depth is used to mean the distance from the surface of the contents 8 of the tank 2.

According to a preferred embodiment, the movement means 22 comprise a cable 19 for moving the sampling means 7 and a cable winding system 20.

More specifically, the sampling means 7 comprise an element 34 for fastening the cable 19.

The cable winding system 20 is configured to indicate the length of the cable 19 used to position the sampling means 7 at a predetermined depth inside the tank 2.

It should be noted that by means of the positioning means 21 and the movement means 22 it is possible to probe the contents 8 of the tank 2 in different sampling 2a areas and, relative to each sampling area 2a, according to depths which are different to each other or according to one or more levels which are different to each other relative to the bottom wall 4 of the tank 2.

Advantageously, the possibility of positioning the sampling means 7 in different sampling areas 2a and, relative to each sampling area 2a, according to different depths, makes it possible to measure the thickness of any layer 44 of fibrous material or any layer 9 of solid material or any layer 45 of muddy material.

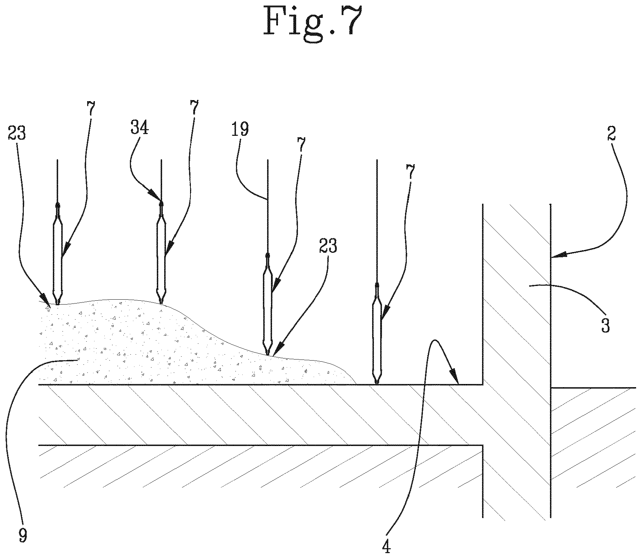

In order to detect any presence of a layer 9 of solid material deposited on the bottom wall 4 of the tank 2, the sampling means 7 are positioned by means of the positioning means 21 at a predetermined sampling area 2a and, subsequently, lowered into the contents 8 of the tank 2 by the movement means 22 until they make contact with an element 23 obstructing the further lowering, as schematically illustrated in Figure 7.

Once the sampling means 7 make contact with the obstructing element 23 by the movement means 22 it is possible to determine the relative depth of the tank 2.

The term depth relative to the tank 2 detected by the movement means 22 means the distance between the surface of the contents 8 of the tank 2 and the obstructing element 23 with which the sampling means 7 make contact.

More specifically, the relative depth of the tank 2 detected by the movement means 22 is determined by measuring the length of cable 19 necessary for the recovery of sampling means 7 from the position in which they make contact with the obstructing element 23.

The depth of the tank 2 detected by the movement means 22 is compared with the height of the contents 8 of the tank 2, which is a known parameter.

If the depth of the tank 2 measured is equal to the height of the contents 8 of the tank 2, the obstructing element 23 of the sampling means 7 is the bottom wall 4 of the tank 2.

If the depth of the tank 2 measured is less than the height of the contents 8 of the tank 2, the obstructing element 23 of the sampling means 7 is a layer 9 of solid material deposited on the bottom wall 4 of the tank 2.

In the latter condition, the thickness of the layer 9 of solid material is equal to the difference between the height of the contents 8 of the tank 2 and the depth of the tank 2 detected by the movement means 22.

Advantageously, by using the device 1 according to the invention it is possible to obtain a mapping of the bottom wall 4 of the tank 2, sampling in the manner described a plurality of areas 2a of the tank 2.

Advantageously, by mapping the bottom wall 4 of the tank 2 an estimate of the fermenting volume of the tank 2 is obtained.

According to the invention, the sampling means 7 are configured to pass from a non-operating configuration to an operating configuration for picking up and retaining a sample 24 of the contents 8 of the tank 2.

The sampling means 7 can be operated from the outside to pass from the non-operating configuration to the operating configuration, once they are positioned in the predetermined sampling area 2a and at a predetermined depth of the tank 2, below the surface of the contents 8 of the tank 2, or height relative to the bottom wall 4.

The sampling means 7 extend mainly along a longitudinal axis 7a.

More specifically, the sampling means 7 comprise a supporting element 39 extending mainly along the longitudinal axis 7a.

The sampling means 7 comprise at least a first body 25 and a second body 26 relatively movable relative to the first body 25.

The first body 25 is fixed along the longitudinal axis 7a.

The second body 26 is movable along the longitudinal axis 7a.

More specifically, the second body 26 runs along the supporting element 39 which defines a guide element of the second body 26.

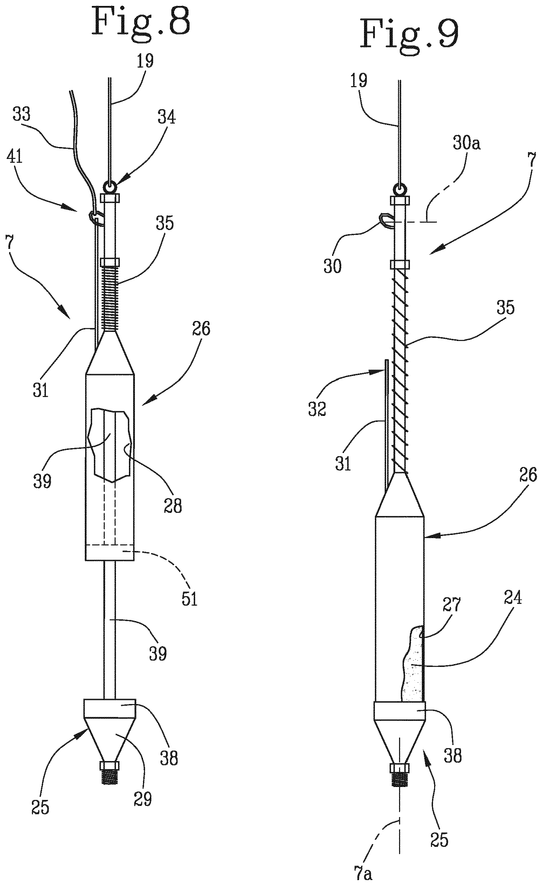

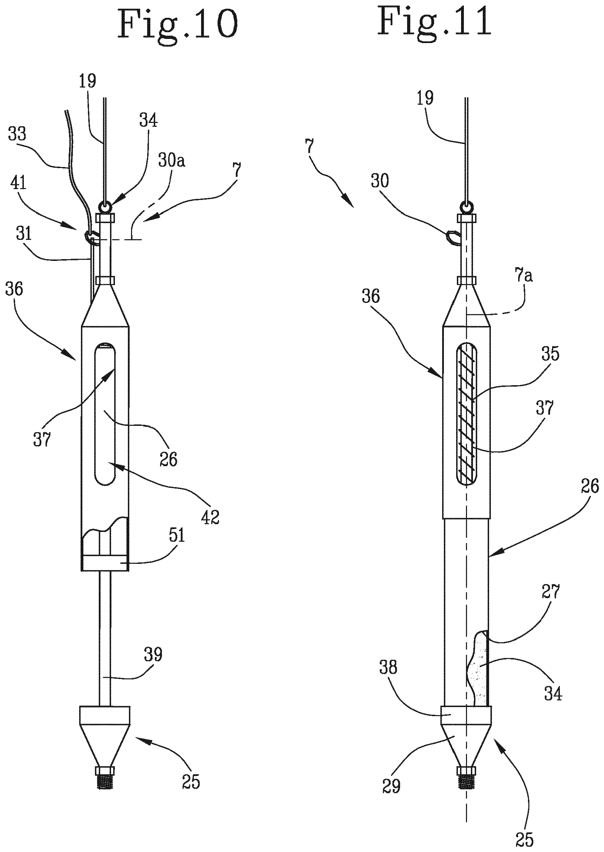

The second body 26 is movable from the non-operating configuration to the operating configuration at which it comes into contact with the first body 25 forming a closed chamber 27 for housing and retaining the sample 24 of the contents 8 of the tank 2, as illustrated in Figures 9 and 11.

The second body 26 has an inner cavity 28 which defines the above-mentioned closed chamber 27 once contact is made with the first body 25. Preferably, the second body 26 has a cylindrical shape.

The first body 25 comprises a stop element 38 of the second body 26.

In the operating configuration, the second body 26 makes contact with the contact element 38 which defines the closing element of the inner cavity 28.

The supporting element 39 comprises an element 51 for closing the inner cavity 28 of the second body 26 at the non-operating configuration of the second body 26.

The closing element 51 is fixed relative to the second body 26, in this way the closing element 51 prevents the contamination of the inner cavity 28 of the second body 26 during the lowering of the sampling means 7 in the contents 8 of the tank 2.

During the passage from the non-operating configuration to the operating configuration, the inner cavity 28 is in communication with the outside for picking up the contents 8 of the tank 2.

The first body 25 is positioned at one end of the supporting element 39.

At the other end of the supporting element 39 is positioned the fastening element 34 of the cable 19.

The first body 25 comprises a ballast 29 of the sampling means 7 in order to favour the immersion in the contents 8 of the tank 2.

Preferably, the ballast 29 comprises lead.

Preferably, the ballast 29 has a conical shape.

Should it be necessary increase the weight of the sampling means 7, further weights, not illustrated, can be interposed between the contact element 38 and the ballast 29.

The sampling means 7 comprise means 41 for triggering the movement of the second body 26 relative to the first body 25 to pass from the non-operating configuration to the operating configuration.

The triggering means 41 are configured to be activated from the outside of the tank 2.

The triggering means 41 comprise an element 30 for releasing the second body 26 for passing from the non-operating configuration to the operating configuration.

The release element 30 is rotatably connected to the supporting element 39 in such a way as to rotate about a relative axis of rotation 30a, at right angles to the axis 7a of longitudinal extension of the supporting element 39.

The triggering means 41 comprise a respective cable 33 for actuating the release element 30 for releasing the second body 26.

At the non-operating configuration, the release element 30 is configured for holding the second body 26, as illustrated in Figures 8 and 10.

More specifically, the second body 26 comprises a rod 31 having an eyelet 32 in which the release element 30 is engaged.

The actuation of the release element 30, in particular by using the drive cable 33, involves the release of the second body 26 which passes from the non-operating configuration to the operating configuration.

The profile of the ring 32 is configured to facilitate its escape from the release element 30 during the operation for releasing the second body 26. The rotation of the release element 30 about the relative axis 30a further favours the escape of the ring 32 from the release element 30.

The triggering means 41 comprise a spring 35 for pushing the second body 26 from the non-operating configuration to the operating configuration.

The spring 35 exerts the pushing action along the direction of the axis 7a of longitudinal extension of the supporting element 39 towards the first body 25.

In the non-operating configuration, the action of the spring 35 is opposed by the action for retaining the second body 26 by the release element 30. Advantageously, the spring 35 for pushing the second body 26 facilitates the disengagement of the ring 32 from the release element 30 during the release operation of the second body 26.

In the operating configuration, the spring 35 for pushing the second body 26 advantageously keeps the second body 26 in contact with the first body 25 in order to retain the sample 24 picked up in the closed chamber 27, avoiding unwanted escape.

The possibility of picking samples 24 from the contents 8 of the tank 2 allows the analysis of the biological state of the contents of the tank 2.

It should be noted that the samples 24 can be picked up both in different sampling zones 2a and at different positions, separate from each other, along the vertical relative to the bottom wall 4 of the tank 2.

This advantageously makes it possible to detect any layers 45 of muddy material or any layers 44 of fibrous material, determining the thickness.

In effect, if the presence of muddy material is found by sampling, it is possible to probe a plurality of positions, different from each other, along the vertical relative to the bottom wall 4 of the tank 2 and pick up a respective sample 24 for each position.

The thickness of the layer 45 of muddy material is determined by the distance between the maximum depth and the minimum depth, relative to the surface of the contents 8 of the tank 2, at which a respective sample 24 of muddy material is picked up.

If the presence of fibrous material is found by sampling, it is possible to probe a plurality of positions, different from each other, along the vertical relative to the bottom wall 4 of the tank 2 and pick up a respective sample 24 for each position.

The thickness of the layer 44 of fibrous material is determined by the distance between the maximum depth and the minimum depth, relative to the surface of the contents 8 of the tank 2, at which a respective sample 24 of fibrous material is picked up.

In a variant embodiment schematically illustrated in Figures 10 and 11, the sampling means 7 comprise a third body 36 for housing at least part of the second body 26.

The third body 36 has a chamber 42 for housing at least part of the second body 26.

In other words, the third body 36 defines an outer jacket of the second body 26.

According to this variant embodiment, the pushing spring 35 of the second body 26 is positioned in the chamber 42 of the third body 36.

The third body 36 is relatively fixed relative to the movement of the second body 26.

More specifically, in the non-operating configuration of the second body 26, it is positioned inside the chamber of the third body 36. In the operating configuration of the second body 26, it is positioned at least partly outside the chamber 42 of the third body 36.

Preferably, the third body 36 has one or more through openings 37 in communication with the chamber 42 of the third body 36 to facilitate the escape of any contents 8 accumulated inside of it in the operating configuration of the second body 26.

Advantageously, the device 1 according to the invention allows the need for emptying a tank 2 to be assessed, to return it to the effective work volume.

Advantageously, by analysing samples 24 of the contents 8 of the tank 2 is possible to estimate the emptying costs, the time required and the tools to be adopted.

Advantageously, by analysing samples 24 of the contents 8 of the tank 2 it is possible to assess the efficiency of any mixing elements positioned in the tank 2.

Advantageously, the device 1 according to the invention is not constrained to the dimensions of the tank 2.

Advantageously, the sampling means 7 allow the biological state of the contents of the tank 2 to be assessed in a predetermined sampling zone 2a.

## Claims

1. A device for probing the contents of tanks of anaerobic digestion systems, comprising means (7) for sampling the contents (8) of the tank (2) which can be introduced into and removed from the inside of the tank (2), and means (22) for moving the sampling means (7) which are configured to move the sampling means (7) along a vertical direction which is orthogonal to the bottom wall (4) of the tank (2);
the device (1) comprising means (21) for positioning the sampling means (7) which are configured to position the sampling means (7) inside the tank (2) in a predetermined sampling area (2a), in particular relative to the perimeter of the tank (2) itself;
**characterised in that** it comprises means (6) for installing the device (1) which are configured to connect the device (1) to the tank (2) to be inspected; the installation means (6) being configured to be positioned in a respective through opening (5) positioned on the lateral wall (3) of the tank (2) or on the upper wall (43) of the tank (2).

2. The device according to claim 1, being **characterised in that** the movement means (22) are configured to detect the depth of the sampling means (7) inside the tank (2), in particular relative to the surface of the contents (8) of the tank (2).

3. The device according to claim 1, being **characterised in that** the positioning means (21) can be introduced into and removed from the inside of the tank (2) using the installation means (6).

4. The device according to preceding claims, **characterised in that** the positioning means (21) comprise at least one element (16) for guiding the sampling means (7) whose extension determines the positioning of the sampling means (7) inside the tank (2) relative to the through opening (5) housing the installation means (6) of the device (1); in particular, the sampling means (7) can be introduced into and removed from the inside of the guide element (16) in order to be inserted into and removed from the contents (8) of the tank (2).

5. The device according to claim 4, being **characterised in that** the positioning means (21) comprise at least one floating element (18) connected to a respective portion (16a) of the guide element (16), the floating element (18) being designed to be inserted into the tank (2).

6. The device according to preceding claims, being **characterised in that** the positioning means (21) comprise means (12) for orienting the sampling means (7) inside the tank (2); the orientation means (12) positioning the sampling means (7) in sampling areas (2a) of the tank (2) relative to a fixed reference element (14a).

7. The device according to claim 6, being **characterised in that** the orientation means (12) comprise an element (14) which oscillates around a main axis of rotation (14a) from a first end position to a second end position which delimit a sampling area (15) of the tank (2).

8. The device according to claim 6 or 7, being **characterised in that** the installation means (6) have a seat (13) for housing at least part of the orientation means (12).

9. The device according to any one of the preceding claims, being **characterised in that** it is possible, using the positioning means (21) and the movement means (22), to position the sampling means (7) in at least one sampling area (2a) and relative to this sampling area (2a), to position the sampling means (7) according to one or more positions, which are separate from each other, along the vertical relative to the bottom wall (4) of the tank (2).

10. The device according to any one of the preceding claims, being **characterised in that** the sampling means (7) are configured to pass from a non-operating configuration to an operating configuration at which they pick and retain a sample (24) of the contents (8) the tank (2).

11. The device according to any one of the preceding claims, being **characterised in that** the sampling means (7) can be operated from the outside to pass from a non-operating configuration to an operating configuration at which they pick and retain a sample (24) of the contents (8) of the tank (2) once they are positioned in a determined sampling area (2a) and according to a predetermined depth inside the tank (2).

12. The device according to any one of the preceding claims, being **characterised in that** the sampling means (7) comprise at least a first body (25) and a second body (26), which is movable relative to the first body (25) from a non-operating configuration to an operating configuration at which it comes into contact with the first body (25), thus forming a closed chamber (27) for housing and retaining a sample (24) of the contents (8) of the tank (2).

13. The device according to claim 12, being **characterised in that** the sampling means (7) comprise a third body (36) for housing at least part of the second body (26); the third body (36) being relatively fixed relative to the second body (26).

14. The device according to claim 12 or 13, being **characterised in that** it comprises means (41) for triggering the movement of the second body (26) relative to the first body (25) to pass from the non-operating configuration to the operating configuration; the triggering means (41) comprising an element (30) for releasing the second body (26) and a spring (35) for pushing the second body (26) to pass from the non-operating configuration to the operating configuration.

15. A method for probing the contents of tanks of anaerobic digestion systems using the device (1) according to any one of claims 1 to 14, comprising the steps of positioning the sampling means (7) at a predetermined sampling area (2a) and then lowering the sampling means (7) into the contents (8) of the tank (2) until they make contact with an element (23) for obstructing the further lowering of them; detecting the relative depth of the tank (2) as the distance between the surface of the contents (8) of the tank (2) and the contact position with the obstruction element (23); comparing the relative depth of the tank (2) with the height of the contents (8) of the tank (2).

16. The method for probing the contents of tanks of anaerobic digestion systems using the device (1) according to any one of claims 1 to 14, comprising the steps of picking samples (24) from the contents (8) of the tank (2) using the sampling means (7) at a predetermined sampling area (2a) and at several positions which are separate from each other, along the vertical relative to the bottom wall (4) of the tank (2); determining the maximum depth and the minimum depth relative to the surface of the contents (8) of the tank (2), at which a respective sample (24) of fibrous or muddy material is picked; deriving the difference between the maximum depth and the minimum depth determined.

## Patentansprüche

1. Vorrichtung zum Prüfen des Inhalts von Tanks anaerober Vergärungssysteme, umfassend Mittel (7) zur Probenahme des Inhalts (8) des Tanks (2), der in das Innere des Tanks (2) eingeführt und aus diesem entfernt werden kann, und Mittel (22) zum Bewegen der Probenahmemittel (7), die konfiguriert sind, um die Probenahmemittel (7) entlang einer vertikalen Richtung zu bewegen, die senkrecht zur Bodenwand (4) des Tanks (2) verläuft; wobei die Vorrichtung (1) Mittel (21) zum Positionieren der Probenahmemittel (7) umfasst, die konfiguriert sind, um die Probenahmemittel (7) innerhalb des Tanks (2) in einem vorbestimmten Probenahmebereich (2a), insbesondere relativ zum Umfang des Tanks (2), zu positionieren; **dadurch gekennzeichnet, dass** sie Mittel (6) zum Installieren der Vorrichtung (1) umfasst, die konfiguriert sind, um die Vorrichtung (1) mit dem zu inspizierenden Tank (2) zu verbinden; wobei die Installationsmittel (6) konfiguriert sind, sodass sie in einer jeweiligen Durchgangsöffnung (5) positioniert werden, die an der Seitenwand (3) des Tanks (2) oder auf der oberen Wand (43) des Tanks (2) positioniert ist.

2. Vorrichtung nach Anspruch 1, **dadurch gekennzeichnet, dass** die Bewegungsmittel (22) so konfiguriert sind, dass sie die Tiefe der Probenahmemittel (7) innerhalb des Tanks (2), insbesondere relativ zur Oberfläche des Inhalts (8) des Tanks (2), erfassen.

3. Vorrichtung nach Anspruch 1, **dadurch gekennzeichnet, dass** die Positionierungsmittel (21) unter Verwendung der Installationsmittel (6) in das Innere des Tanks (2) eingeführt und aus diesem entfernt werden können.

4. Vorrichtung nach vorhergehenden Ansprüchen, **dadurch gekennzeichnet, dass** die Positionierungsmittel (21) mindestens ein Element (16) zum Führen der Probenahmemittel (7) umfassen, dessen Ausdehnung die Positionierung der Probenahmemittel (7) innerhalb des Tanks (2) relativ zur Durchgangsöffnung (5), die die Installationsmittel (6) der Vorrichtung (1) aufnimmt, bewirkt; insbesondere können die Probenahmemittel (7) in das Innere des Führungselements (16) eingeführt und von diesem entfernt werden, um in den Inhalt (8) des Tanks (2) eingesetzt und aus diesem entfernt zu werden.

5. Vorrichtung nach Anspruch 4, **dadurch gekennzeichnet, dass** die Positionierungsmittel (21) mindestens ein schwimmendes Element (18) umfassen, das mit einem jeweiligen Abschnitt (16a) des Führungselements (16) verbunden ist, wobei das schwimmende Element (18) ausgelegt ist, um in den Tank (2) eingesetzt zu werden.

6. Vorrichtung nach vorhergehenden Ansprüchen, **dadurch gekennzeichnet, dass** die Positionierungsmittel (21) Mittel (12) zum Ausrichten der Probenahmemittel (7) innerhalb des Tanks (2) umfassen; wobei die Orientierungsmittel (12) die Probenahmemittel (7) in Probenahmebereichen (2a) des Tanks (2) relativ zu einem fixierten Bezugselement (14a) positionieren.

7. Vorrichtung nach Anspruch 6, **dadurch gekennzeichnet, dass** die Orientierungsmittel (12) ein Element (14) umfassen, das um eine Hauptdrehachse (14a) von einer ersten Endposition zu einer zweiten Endposition schwingt, die einen Probenahmebereich (15) des Tanks (2) begrenzen.

8. Vorrichtung nach Anspruch 6 oder 7, **dadurch gekennzeichnet, dass** die Installationsmittel (6) einen Sitz (13) aufweisen, um mindestens einen Teil der Orientierungsmittel (12) aufzunehmen.

9. Vorrichtung nach einem der vorhergehenden Ansprüche, **dadurch gekennzeichnet, dass** es unter Verwendung der Positionierungsmittel (21) und der Bewegungsmittel (22) möglich ist, die Probenahmemittel (7) in mindestens einem Probenahmebereich (2a) zu positionieren und relativ zu diesem Probenahmebereich (2a), um die Probenahmemittel (7) gemäß einer oder mehreren voneinander getrennten Positionen entlang der Vertikalen relativ zur Bodenwand (4) des Tanks (2) zu positionieren.

10. Vorrichtung nach einem der vorhergehenden Ansprüche, **dadurch gekennzeichnet, dass** die Probenahmemittel (7) so konfiguriert sind, dass sie von einer Nichtbetriebskonfiguration zu einer Betriebskonfiguration übergehen, bei der sie eine Probe (24) des Inhalts (8) des Tanks (2) entnehmen und halten.

11. Vorrichtung nach einem der vorhergehenden Ansprüche, **dadurch gekennzeichnet, dass** die Probenahmemittel (7) von außen betrieben werden können, um von einer Nichtbetriebskonfiguration zu einer Betriebskonfiguration überzugehen, bei der sie eine Probe (24) des Inhalts (8) des Tanks (2) entnehmen und halten, sobald sie in einem bestimmten Probenahmebereich (2a) und gemäß einer vorbestimmten Tiefe innerhalb des Tanks (2) positioniert werden.

12. Vorrichtung nach einem der vorhergehenden Ansprüche, **dadurch gekennzeichnet, dass** die Probenahmemittel (7) mindestens einen ersten Körper (25) und einen zweiten Körper (26) umfassen, der relativ zu dem ersten Körper (25) von einer Nichtbetriebskonfiguration zu einer Betriebskonfiguration beweglich ist, bei der er mit dem ersten Körper (25) in Kontakt kommt, wodurch eine geschlossene Kammer (27) zum Aufnehmen und Halten einer Probe (24) des Inhalts (8) des Tanks (2) geformt wird.

13. Vorrichtung nach Anspruch 12, **dadurch gekennzeichnet, dass** die Probenahmemittel (7) einen dritten Körper (36) zum Aufnehmen mindestens eines Teils des zweiten Körpers (26) umfassen; wobei der dritte Körper (36) relativ zum zweiten Körper (26) fixiert ist.

14. Vorrichtung nach Anspruch 12 oder 13, **dadurch gekennzeichnet, dass** sie Mittel (41) zum Auslösen der Bewegung des zweiten Körpers (26) relativ zum ersten Körper (25) zum Übergang von der Nichtbetriebskonfiguration zur Betriebskonfiguration umfasst; wobei die Auslösemittel (41) ein Element (30) zum Lösen des zweiten Körpers (26) und eine Feder (35) zum Drücken des zweiten Körpers (26) umfassen, um von der Nichtbetriebskonfiguration zur Betriebskonfiguration überzugehen.

15. Verfahren zum Prüfen des Inhalts von Tanks anaerober Vergärungssysteme unter Verwendung der Vorrichtung (1) nach einem der Ansprüche 1 bis 14, umfassend die Schritte zum Positionieren der Probenahmemittel (7) an einem vorbestimmten Probenahmebereich (2a) und anschließend zum Absenken der Probenahmemittel (7) in den Inhalt (8) des Tanks (2), bis sie mit einem Element (23) ein Kontakt herstellen, um deren weiteres Absenken zu behindern; Erfassen der relativen Tiefe des Tanks (2) als Abstand zwischen der Oberfläche des Inhalts (8) des Tanks (2) und der Kontaktposition mit dem Hinderniselement (23); Vergleichen der relativen Tiefe des Tanks (2) mit der Höhe des Inhalts (8) des Tanks (2).

16. Verfahren zum Prüfen des Inhalts von Tanks anaerober Vergärungssysteme unter Verwendung der Vorrichtung (1) nach einem der Ansprüche 1 bis 14, umfassend die Schritte zum Entnehmen von Proben (24) aus dem Inhalt (8) des Tanks (2) unter Verwendung der Probenahmemittel (7) an einem vorbestimmten Probenahmebereich (2a) und an mehreren voneinander getrennten Positionen entlang der Vertikalen relativ zur Bodenwand (4) des Tanks (2); Bestimmen der maximalen Tiefe und der minimalen Tiefe relativ zur Oberfläche des Inhalts (8) des Tanks (2), bei der eine jeweilige Probe (24) aus faserigem oder schlammigem Material entnommen wird; Ableiten der Differenz zwischen der bestimmten maximalen und der minimalen Tiefe.

## Revendications

1. Dispositif pour tester le contenu de cuves de systèmes de digestion anaérobie, comprenant des moyens (7) pour échantillonner le contenu (8) de la cuve (2) qui peut être introduit dans et retiré de l'intérieur de la cuve (2), et des moyens (22) pour déplacer les moyens d'échantillonnage (7) qui sont configurés pour déplacer les moyens d'échantillonnage (7) le long d'une direction verticale qui est orthogonale à la paroi de fond (4) de la cuve (2) ;
le dispositif (1) comprenant des moyens (21) pour positionner les moyens d'échantillonnage (7) qui sont configurés pour positionner les moyens d'échantillonnage (7) à l'intérieur de la cuve (2) dans une zone d'échantillonnage prédéterminée (2a), en particulier par rapport au périmètre de la cuve (2) elle-même ;
**caractérisé en ce qu'**il comprend des moyens (6) pour installer le dispositif (1) qui sont configurés pour relier le dispositif (1) à la cuve (2) à inspecter ; les moyens d'installation (6) étant configurés pour être positionnés dans une ouverture traversante respective (5) positionnée sur la paroi latérale (3) de la cuve (2) ou sur la paroi supérieure (43) de la cuve (2).

2. Dispositif selon la revendication 1, **caractérisé en ce que** les moyens de déplacement (22) sont configurés pour détecter la profondeur des moyens d'échantillonnage (7) à l'intérieur de la cuve (2), en particulier par rapport à la surface du contenu (8) de la cuve (2).

3. Dispositif selon la revendication 1, **caractérisé en ce que** les moyens de positionnement (21) peuvent être introduits dans et retirés de l'intérieur de la cuve (2) en utilisant les moyens d'installation (6).

4. Dispositif selon les revendications précédentes, **caractérisé en ce que** les moyens de positionnement (21) comprennent au moins un élément (16) pour guider les moyens d'échantillonnage (7) dont l'extension détermine le positionnement des moyens d'échantillonnage (7) à l'intérieur de la cuve (2) par rapport à l'ouverture traversante (5) accueillant les moyens d'installation (6) du dispositif (1) ; en particulier, les moyens d'échantillonnage (7) peuvent être introduits dans et retirés de l'intérieur de l'élément de guidage (16) afin d'être insérés dans et retirés du contenu (8) de la cuve (2) .

5. Dispositif selon la revendication 4, **caractérisé en ce que** les moyens de positionnement (21) comprennent au moins un élément flottant (18) relié à une partie respective (16a) de l'élément de guidage (16), l'élément flottant (18) étant conçu pour être inséré dans la cuve (2) .

6. Dispositif selon les revendications précédentes, **caractérisé en ce que** les moyens de positionnement (21) comprennent des moyens (12) pour orienter les moyens d'échantillonnage (7) à l'intérieur de la cuve (2) ; les moyens d'orientation (12) positionnant les moyens d'échantillonnage (7) dans des zones d'échantillonnage (2a) de la cuve (2) par rapport à un élément de référence fixe (14a).

7. Dispositif selon la revendication 6, **caractérisé en ce que** les moyens d'orientation (12) comprennent un élément (14) qui oscille autour d'un axe principal de rotation (14a) d'une première position d'extrémité à une deuxième position d'extrémité qui délimitent une zone d'échantillonnage (15) de la cuve (2).

8. Dispositif selon la revendication 6 ou 7, **caractérisé en ce que** les moyens d'installation (6) ont un siège (13) pour accueillir au moins une partie des moyens d'orientation (12).

9. Dispositif selon l'une quelconque des revendications précédentes, **caractérisé en ce qu'**il est possible, en utilisant les moyens de positionnement (21) et les moyens de déplacement (22), de positionner les moyens d'échantillonnage (7) dans au moins une zone d'échantillonnage (2a) et par rapport à cette zone d'échantillonnage (2a), de positionner les moyens d'échantillonnage (7) selon une ou plusieurs positions, séparées les unes des autres, le long de la verticale par rapport à la paroi de fond (4) de la cuve (2).

10. Dispositif selon l'une quelconque des revendications précédentes, **caractérisé en ce que** les moyens d'échantillonnage (7) sont configurés pour passer d'une configuration non opérationnelle à une configuration opérationnelle dans laquelle ils prélèvent et maintiennent un échantillon (24) du contenu (8) de la cuve (2).

11. Dispositif selon l'une quelconque des revendications précédentes, **caractérisé en ce que** les moyens d'échantillonnage (7) peuvent être actionnés de l'extérieur pour passer d'une configuration non opérationnelle à une configuration opérationnelle dans laquelle ils prélèvent et retiennent un échantillon (24) du contenu (8) de la cuve (2) une fois qu'ils sont positionnés dans une zone d'échantillonnage déterminée (2a) et selon une profondeur prédéterminée à l'intérieur de la cuve (2).

12. Dispositif selon l'une quelconque des revendications précédentes, **caractérisé en ce que** les moyens d'échantillonnage (7) comprennent au moins un premier corps (25) et un second corps (26), qui est mobile par rapport au premier corps (25) d'une configuration non opérationnelle à une configuration opérationnelle dans laquelle il entre en contact avec le premier corps (25), formant ainsi une chambre fermée (27) pour accueillir et retenir un échantillon (24) du contenu (8) de la cuve (2) .

13. Dispositif selon la revendication 12, **caractérisé en ce que** les moyens d'échantillonnage (7) comprennent un troisième corps (36) pour accueillir au moins une partie du deuxième corps (26) ; le troisième corps (36) étant relativement fixe par rapport au deuxième corps (26).

14. Dispositif selon la revendication 12 ou 13, **caractérisé en ce qu'**il comprend des moyens (41) pour déclencher le mouvement du deuxième corps (26) par rapport au premier corps (25) pour passer de la configuration non opérationnelle à la configuration opérationnelle ; les moyens de déclenchement (41) comprenant un élément (30) pour libérer le deuxième corps (26) et un ressort (35) pour pousser le deuxième corps (26) à passer de la configuration non opérationnelle à la configuration opérationnelle.

15. Procédé pour tester le contenu de cuves de systèmes de digestion anaérobie utilisant le dispositif (1) selon l'une quelconque des revendications 1 à 14, comprenant les étapes de positionner les moyens d'échantillonnage (7) dans une zone d'échantillonnage prédéterminée (2a) et d'abaisser ensuite les moyens d'échantillonnage (7) dans le contenu (8) de la cuve (2) jusqu'à ce qu'ils entrent en contact avec un élément (23) pour empêcher la poursuite de leur abaissement ; de détecter la profondeur relative de la cuve (2) comme étant la distance entre la surface du contenu (8) de la cuve (2) et la position de contact avec l'élément d'obstruction (23) ; de comparer la profondeur relative de la cuve (2) avec la hauteur du contenu (8) de la cuve (2).

16. Procédé pour tester le contenu des cuves de systèmes de digestion anaérobie utilisant le dispositif (1) selon l'une quelconque des revendications 1 à 14, comprenant les étapes de prélever des échantillons (24) du contenu (8) de la cuve (2) en utilisant les moyens d'échantillonnage (7) dans une zone d'échantillonnage prédéterminée (2a) et en plusieurs positions qui sont séparées les unes des autres, le long de la verticale par rapport à la paroi de fond (4) de la cuve (2) ; de déterminer la profondeur maximale et la profondeur minimale par rapport à la surface du contenu (8) de la cuve (2), dans laquelle un échantillon respectif (24) de matière fibreuse ou boueuse est prélevé ; de dériver la différence entre la profondeur maximale et la profondeur minimale déterminée.
